# EUROPÄISCHE PATENTSCHRIFT

(11) **EP 0 412 549 B1**
(45) Veröffentlichungstag und Bekanntmachung des Hinweises auf die Patenterteilung: **08.06.1994**
(21) Anmeldenummer: 90115323.9
(22) Anmeldetag: 09.08.1990
(51) Int. Cl.: A61F 13/15

(54) **Wegwerfkleidungsstück**
Disposable garment
Vêtement jetable

(30) Priorität: 10.08.1989 JP 209203/89; 19.09.1989 JP 242711/89
(43) Veröffentlichungstag der Anmeldung: 13.02.1991
(73) Patentinhaber: UNI-CHARM CORPORATION, Kawanoe-shi Ehime-ken (JP)
(72) Erfinder: Iguaue, Takamitsu, Kawanoe-shi, Ehime-ken (JP); Kuramoto, Katsuhide, Kawanoe-shi, Ehime-ken (JP); Nomura, Hironori, Iyomishima-shi, Ehime-ken (JP); Sasaki, Tohru, Kawanoe-shi, Ehime-ken (JP)
(74) Vertreter: Sperling, Rüdiger, Dipl.-Ing.

(56) Entgegenhaltungen:
- EP-A- 0 048 011
- US-A- 4 655 760
- US-A- 4 731 066
- US-A- 4 842 596
- US-A- 4 887 602

## Beschreibung

Die vorliegende Erfindung betrifft Wegwerfunterwäsche, insbesondere solche Wegwerfunterwäsch, wie beispielsweise Windein vom offenen Typ, Wegwerfwindelhosen oder Slips (einschl. solcher für Inkontinenzler) oder sogenannte Kinder-Trainingsunterhosen.

Es ist bekannt, derartige Unterwäsche beispielsweise in Form von Unterhosen oder Slips, insbesondere solche Kinder-Trainingsunterhosen mit geeigneten elastischen Teilen um die jeweiligen Beinöffnungen, wie auch um die Taillenöffnung herum auszustatten und einen Saugkern sandwichartig zwischen einer wasserdurchlässigen und einer unteren Lage anzuordnen.

Aus der US-A-4 842 596 ist eine Windelhose gemäß Oberbegriff des Hauptanspruchs bekannt. Bei dieser Windelhose sind an den Beinausschnitten jeweils breite elastische Bänder vorgesehen, die am vorderen Bereich der Windelhose beginnen an den jeweiligen Beinausschnitten entlanglaufen bis in den hinteren Bereich der Windelhose verlaufen. Diese elastischen Bänder sind in geeigneter Weise an der unteren Lage befestigt.

Da diese elastischen Bänder jeweils im Bauchbereich bzw. im Gesäßbereich auslaufen, ist eine komplette Umschließung des Beins des Trägers nicht gegeben.

Der Erfindung liegt die Aufgabe zugrunde, bei einer Windelhose des genannten Typs unter Beibehaltung der Luftdurchlässigkeit die Hose insbesondere im Beinbereich so zu gestalten, daß mögliche Leckagen der flüssigen Ausscheidung vermieden wird.

Ein Hauptziel der Erfindung liegt darin, die gegensätzlichen Verhältnisse optimal zu lösen, die gegeben sind zum einen durch die Tatsache, daß es bevorzugt ist, obere und untere Lagen mit guter Luftdurchlässigkeit zu verwenden, wie es üblicherweise für Wegwerfunterwäsche bisher geschehen ist und der Tatsache, daß es unerwünscht ist, Faservlies zumindest für die untere Lage zu verwenden, um mögliche Leckagen der flüssigen Ausscheidungen zu minimieren.

Wenn sich die Sperrlage zwischen der oberen und der unteren Lage im wesentlichen über den gesamten Bereich erstreckt, dann wird es bevorzugt, die Sperrlage entlang beider Seiten der vorderen und rückwärtigen Zonen mit Perforationen zu versehen, die als Luftöffnungen dienen.

Bei Unterwäsche, die entsprechend der oben erwähnten Erfindung aufgebaut ist, dehnen und ziehen sich die obere und untere Lage gemeinsam zusammen, so, wie sich der Körper des Trägers bewegt. Die Sperrlage dient als eine Sperre, die verhindert, daß eine beträchtliche Menge der Körperflüssigkeit in Richtung der Außenfläche des Wäsche- oder Bekleidungsstücks dringt.

Wenn die Sperrlage auf beiden Seiten in den vorderen und den rückwärtigen Zonen mit als Luftöffnung dienenden Perforationen ausgestattet ist, dann stellen diese Öffnungen selbst dann eine gewünschte Ventilation bereit, wenn die Sperrlage zwischen der vorderen und unteren Lage im wesentlichen über den gesamten Bereich ausgedehnt ist. Wenn die Sperrlage zwischen der oberen und der unteren Lage über einen Bereich ausgedehnt ist, der schmäler ist als der Bereich der oberen und unteren Lage, jedoch größer als der Kern, dann wird die gewünschte Ventilation durch die beiden Seitenbereiche der oberen und unteren Lagen bereitgestellt, die nicht von der Sperrlage belegt sind.

Ist der Kern zwischen der oberen und der unteren Lage sandwichartig eingelegt, dann wird die Menge Körperflüssigkeit, die durch die obere Lage hindurchgetreten ist, von dem Kern aufgesaugt.

### Kurzer Überblick über die Figuren.

In den Figuren ist ein Ausführungsbeispiel der Erfindung dargestellt. Es zeigen:
- Fig. 1: eine perspektivische Gesamtansicht des erfindungsgemäßen Bekleidungsstücks;
- Fig. 2: eine perspektivische Explosionsdarstellung;
- Fig. 3: eine perspektivische Ansicht der unteren Lage;
- Fig. 4: eine perspektivische Ansicht eines Ausführungsbeispiels der elastischen Teile, die zwischen einer unteren Lage und der Sperrlage eingesetzt sind;
- Fig. 5: eine perspektivische Ansicht einer relativ schmalen Sperrlage und
- Fig. 6: eine Schnittdarstellung, die zeigt, wie die untere Lage und die Sperrlage miteinander verbunden sind.

### Bevorzugtes Ausführungsbeispiel der Erfindung.

Im folgenden wird ein bevorzugtes Ausführungsbeispiel der Erfindung unter Bezugnahme auf die begleitenden Zeichnungen beispielhaft beschrieben.

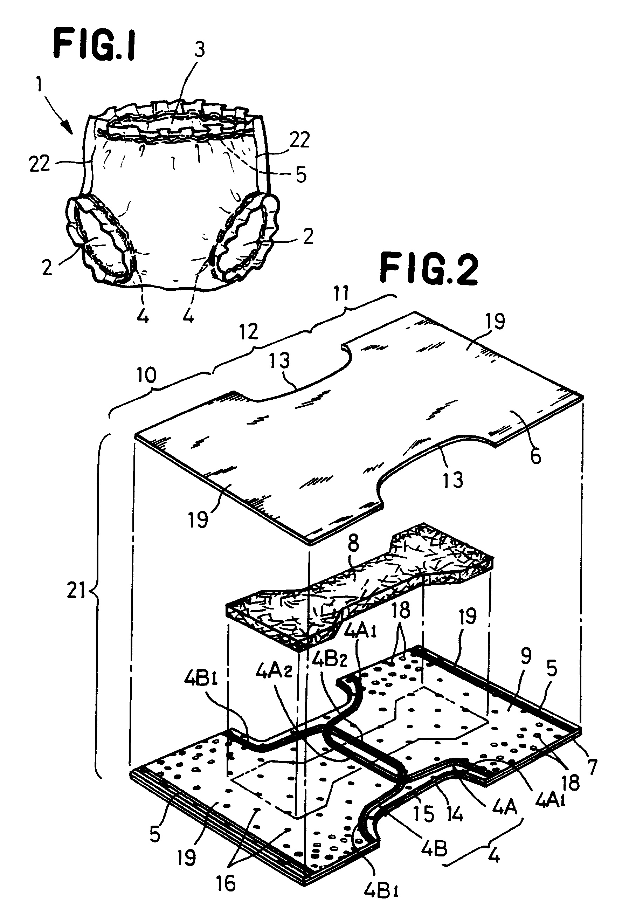

Es wird zunächst Bezug genommen auf Fig. 1. Darin weist ein Kleidungsstück 1 Beinöffnungen 2 und eine Taillenöffnung 3 auf, die jeweils mit elastischen Teilen 4 und 5 ausgestattet sind.

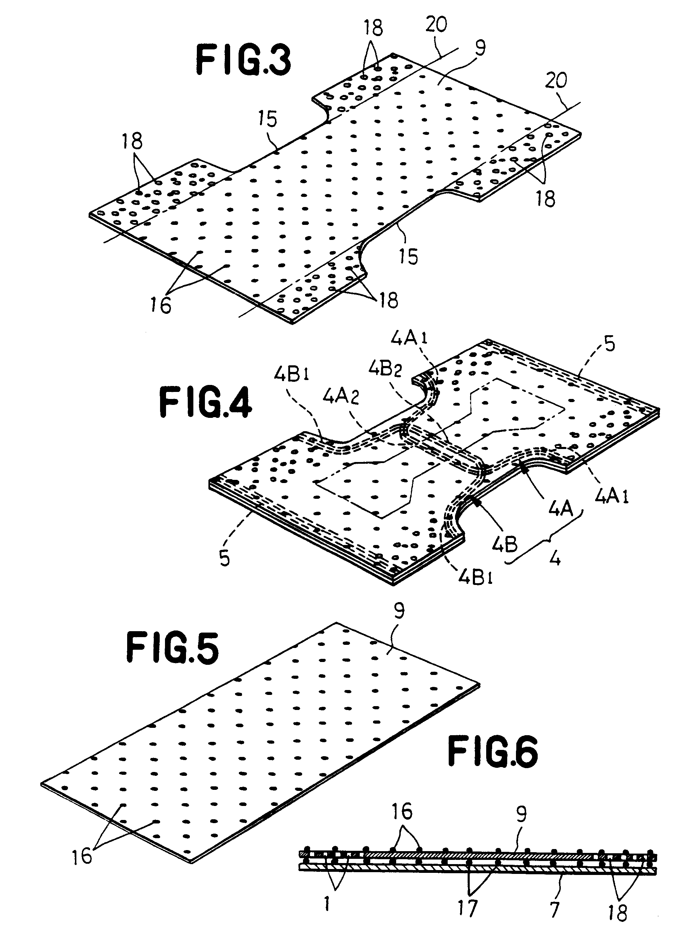

In Fig. 2 ist der Aufbau der Lagen dargestellt. Das Kleidungsstück 1 weist eine elastische wasserdurchlässige obere Lage aus Faservlies auf, eine elastische wasserdurchlässige untere Lage 7 aus dem gleichen Faservlies, einen matten- oder lagenartigen Kern 8, elastische Teile 4,5, die sich um die Beinöffnungen und die Taillenöffnungen herum erstrecken und eine elastische Sperrlage 9, die aus einem elastomeren Film besteht. Die oberen und unteren Lagen 6 und 7 definieren gemeinsam mit der Sperrlage 9 zwischen ihren vorderen und ihren rückwärtigen Zonen 10 und 11 einen Schrittbereich 12, dessen beide Seitenränder mit Ausschnitten 13,14 und 15 ausgestattet sind. Die Sperrlage 9 ist mit der unteren Lage 7 über den gesamten Bereich mittels eines heißschmelzenden Adhesivs oder durch unmittelbares Verschmelzen verbunden. Der Kern 9 ist punktweise mit der Oberfläche der Sperrlage 9 mittels eines heißschmelzenden Klebmittels oder durch Verschweißen verbunden. Das Verkleben des Kerns 8 und der Sperrlage 9 und das Verbinden zwischen der oberen Lage 6 und der Sperrlage 9 kann entweder über eine Vielzahl von punktförmigen Klebmitteln 16 oder mittels einer Vielzahl von intermittierenden Linien jeweils geeigneter Länge erzielt werden. Wie aus Fig. 4 zu ersehen ist, kann das Verkleben zwischen der unteren Lage 7 und der Sperrlage 9 ebenfalls mittels einer Vielzahl von Klebpunkten 17 (s. Fig. 6) oder eine Vielzahl von unterbrochenen Linien jeweils geeigneter Länge durchgeführt werden.

Es wird bevorzugt, daß der Kern 8 nicht an der Sperrlage 9 sondern an der oberen Lage 6 angeklebt ist, und daß der Kern 8, die untere Lage 7 und die Sperrlage 9 jeweils intermittierend miteinander verbunden sind. Auf diese Weise kann die Elastizität der oberen Lage 6, der unteren Lage 7 und der Sperrlage 9 wirksam ausgenutzt werden und es wird vermieden, daß die Aufnahme von Körperfluid durch den Kern 8 erschwert wird, was andernfalls auftreten könnte, wegen teilweisem Aufschwimmen der oberen Lage 6 von dem Kern 8.

Entlang der beiden Seiten an ihren vorderen und rückwärtigen Bereichen 10 und 11 ist die Sperrlage 9 mit feinen Luftdurchlässen 18 versehen (s. Fig. 3). Es versteht sich, daß diese Luftdurchlässe 18 sich auch durch die obere und untere Lage 6,7 erstrecken können.

Die elastischen Teile 4,5 für die Bein- bzw. Taillenöffnungen sind um die herausgeschnittenen Ränder 13 und 15 der oberen Lage und der Sperrlage 9, wie auch entlang der beiden Enden 19 der vorderen und rückwärtigen Zonen 10 und 11 herum angeordnet. Jede der elastischen Beinteile 4 weist ein erstes Teil 4A und ein zweites Teil 4B auf, welche sich an Punkten in der Nähe ihrer einander gegenüberliegenden Enden schneiden; ihre Seitenabschnitte 4A₁, 4B₁, die sich von den jeweiligen Schnittpunkten zu den jeweiligen Enden hin erstrecken, sind mit heißschmelzendem Kleber auf die innere Oberfläche der oberen Lage 6 und/oder der Sperrlage 9 entlang der ausgeschnittenen Ränder 13,15 angeklebt, während ihre Zwischenabschnitte 4A_{2,} 4B₂, zentral unter dem Kern angeordnet sind, ohne mit einer der Lagen verbunden zu sein.

Es versteht sich, daß diese Zwischenabschnitte 4A₂, 4B₂ bei einem anderen Ausführungsbeispiel an einer beliebigen der genannten Lagen befestigt sein können. Jedes der elastischen Teile 5 ist mit einem Kleber heißschmelzenden Typs an der inneren Oberfläche der oberen Lage und/oder der Sperrlage 9 entlang des zugeordneten Endes 19 angeklebt. Die obere Lage 6 und die Sperrlage 9 können, falls erforderlich, durch Ultraschallverschweißung oder Verkleben mit heißschmelzendem Kleber miteinander entlang ihrer äußeren Ränder verbunden sein. Die obere Oberfläche des Kerns 9 kann ebenfalls intermittierend an der oberen Lage 6 angeklebt sein, falls erforderlich.

Wie mit einer Kettenlinie in Fig. 6 dargestellt, ist es auch möglich, die elastischen Teile 4 zwischen der unteren Lage 7 und der Sperrlage 9 anzuordnen und in einem solchen Fall sind die elastischen Teile vorzugsweise an der unteren Lage 7 angeklebt.

Die Sperrlage 9, dargestellt in Fig. 5, besitzt eine Breite, entsprechend derjenigen, die in Fig. 3 mit einem Paar paralleler Kettenlinien 20 dargestellt ist, wobei die Abschnitte, die sich über die jeweiligen Kettenlinien 20 in Fig. 3 hinauserstrecken, in dem in Fig. 5 dargestellten Ausführungsbeispiel weggeschnitten sind. Bei einer solchen Ausführungsform wird die Luftzirkulation durch die beiden- Seitenbereiche der oberen und der unteren Lage sichergestellt, die nicht durch die Sperrlage 9 abgedeckt sind.

Ein auf diese Weise konstruierter Laminataufbau 21 wird entlang eines mittleren Längsabschnitts gefaltet und intermittierend entlang der beiden Seiten Ränder durch Heißschweissen oder Ultraschall-Schweißmittel 27 zu Kleidungsstücken wie in Fig. 1 dargestellt, verschweißt, so daß diese miteinander verklebten Seitenränder manuell ohne Anstrengung aufgezogen werden können. Es versteht sich, daß die erfindungsgemäßen Kleidungsstücke als Endprodukt in Form der Kleidungsstücke der offenen Art hergestellt werden können ohne Heißschmelzmittel oder Ultraschallschweißmittel 22 ausgesetzt zu sein, in einem solchen Fall wird die rückwärtige Zone 11 (s. Fig. 2) entlang beider Seitenabschnitte mit Befestigungsmitteln versehen, wie beispielsweise Bandbefestiger der bekannten Art. Manchmal ist kein Kern 8 vorgesehen, jeweils abhängig von dem besonderen Typ des Kleidungsstücks.

Die obere und die untere Lage 6,7 können aus Faservlies hergestellt sein, das sowohl in der Längs- als auch in der Querrichtung elastisch ist; der Kern 8 kann eine heißpreßgeformte Mischung aus flockiger Pulpe, wasserabsorbierenden Polymerpartikeln und heißschmelzenden Fasern sein; die Sperrlage 9 besteht aus einem elastomeren Film, der sowohl in Längs- als auch in Querrichtung elastisch ist und jedes der elastischen Teile 4,5 kann zumindest einen Faden oder ein Band aus Gummi oder Kunststoffilm enthalten.

Das als Material für die obere und die untere Lage 6,7 verwendete Faservlies wird vorzugsweise aus kardierten, heiß gekräuselten Fasern mit einem Gewicht Pro Flächeneinheit von 25 bis 45 g/m² und einer Feinheit von 0,5 bis 3 d zu einer Bahn geformt und anschließend wird diese Bahn heiß behandelt und zu einer Lage geformt. Bevorzugte Beispiele solcher heiß gekräuselter Fasern sind solche, die von der Fa. Chisso Co., Ltd. in Japan und dem Handelsnamen "EP FASERN" vertrieben werden.

Erfindungsgemäß dient die zwischen der oberen und der unteren Lage, die beide aus elastischem Faservlies hergestellt sind, eingelegte Sperrlage nicht nur dazu, das Auslaufen von flüssigen Ausscheidungen zu verhindern, sondern verbessert auch die Paßform des Kleidungsstücks am Körper des Trägers, da die Sperrlage ebenfalls eine Elastizität aufweist und mit entweder der oberen oder der unteren Lage intermittierend verbunden ist, so daß sie sich integral mit der einen oder anderen Lage dehnt oder zusammenzieht. Selbst wenn die Sperrlage zwischen der oberen und der unteren Lage über die gesamte Fläche dieser Lagen hinweg eingelegt ist, so verhindern die Luftöffnungen, die in der vorderen und rückwärtigen Zone entlang der beiden Seiten der Sperrlage ausgebildet sind, das innere Bekleidungsstück davor, stickig zu werden.

Wenn ein Saugkern vorgesehen ist, dann dient die gute Paßform im Zusammenwirken mit einem Abdichteffekt, der durch die Bein- und Taillenelastikteile bereitgestellt wird, dazu, das Auslaufen von flüssiger Ausscheidung wirksam zuverhindern.

Die Sperrlage ist aus einem elastischen Film hergestellt, der anders als das Faservlies während des Dehnens im wesentlichen frei ist von einer rasch herabgesetzten Kontraktionsspannung, die aufgrund sich lockernder Faserverschlingung und Freisetzen der Faserverklebung auftreten kann, so daß diese Sperrlage sehr wirkungsvoll ist, eine gewünschte Zusammenziehspannung bzw. einen Zug im gesamten Material des Kleidungsstücks aufrechtzuerhalten.

## Patentansprüche

1. Kleidungsstück mit einer elastischen, wasserdurchlässigen oberen Lage (6), einer elastischen wasserdurchlässigen unteren Lage (7) und elastischen Bein- und Taillenteilen (4,5), die entlang beider Seiten im Schrittbereich und den entsprechenden Enden von vorder- und rückwärtigen Zonen angeordnet sind, mit einer elastischen Sperrlage (9) die sandwichartig zwischen der oberen und der unteren Lage (6,7,) angeordnet ist, und intermittierend zumindest an einer der beiden genannten Lagen befestigt ist dadurch **gekennzeichnet**, daß jedes der elastischen Beinteile (4) einen ersten Teilabschnitt (4A) und einen zweiten Teilabschnitt (4B) aufweisen, die sich an Punkten in der Nähe ihrer gegenüberliegenden Enden schneiden und deren Seitenabschnitte (4A₁, 4B₁) sich von den jeweiligen Schnittpunkten zu jeweiligen Enden erstrecken und an der inneren Oberfläche der oberen Lage (6) und/oder der Sperrlage (9) befestigt sind.

2. Kleidungsstück nach Anspruch 1, dadurch **gekennzeichnet** daß Zwischenabschnitte (4A₂ , 4B₂) der elastischen Beinteile in der Mitte unter einem Saugkern (8) angeordnet sind, ohne mit einer der Lagen verbunden zu sein.

3. Kleidungsstück nach Anspruch 1 oder 2, wobei die Sperrlage (9) sandwichartig zwischen der oberen und der unteren Lage (6,7) im wesentlichen über deren gesamte Fläche angeordnet ist, und daß in den vorderen und rückwärtigen Zonen die Sperrlage (9) entlang beider Seitenränder mit Luftöffnungen (16) versehen ist.

4. Kleidungsstück nach mindestens einem der Ansprüche 1 bis 3, wobei die Sperrlage (9) schmäler ist als die Breite der oberen und der unteren Lagen (6,7).

5. Kleidungsstück nach mindestens einem der Ansprüche 1 bis 4, wobei die Sperrlage (9) auf der inneren Oberfläche der unteren Lage (7) befestigt ist, ein Saugkern (8) sandwichartig zwischen der Sperrlage (9) und der oberen Lage (6) angeordnet ist und der Kern intermittierend zumindest an entweder der Sperrlage (9) oder der unteren Lage (7) befestigt ist.

6. Kleidungsstück nach mindestens einem der Ansprüche 1 bis 5, wobei ein Laminataufbau, hergestellt aus der Sperrlage (9), die sandwichartig zwischen der oberen und der unteren Lage (6,7) und intermittierend an einer der beiden Lagen befestigt ist, entlang eines Längsmittelabschnitts gefaltet und entlang der beiden Seitenränder der vorderen und rückwärtigen Zone miteinander verbunden ist, so daß Beinöffnungen und eine Hüftöffnung definiert sind, denen die elastische Teile zugeordnet sind.

7. Kleidungsstück, nach mindestens einem der Ansprüche 1 bis 6, wobei die obere und die untere Lage (6,7) aus einem Faservlies und die Sperrlage (9) aus einem elastomeren Film hergestellt sind.

8. Kleidungsstück nach mindestens einem der Ansprüche 2 bis 7, wobei der Saugkern (8) eine Mischung aus heißgeschmolzener flockiger Pulpe, wasserabsorbierenden Polymerpartikeln und schweißbaren Fasern aufweist.

## Claims

1. Garment with an elastic, water-permeable outer layer (6), and elastic, water-permeable inner layer (7) and elastic leg and waist portions (4, 5) which are disposed along both sides in the crotch area and the corresponding ends of forward and rear zones, with an elastic barrier layer (9) disposed sandwich fashion between the outer and inner layers (6, 7) and attached at intermittent points to at least one of the two named layers, characterised in that each of the elastic leg portions (4) has a first part section (4A) and a second part section (4B) which intersect at points in the vicinity of their oppositely located ends, and whose lateral portions (4A₁, 4B₁) extend from the respective points of intersection to respective ends, and are attached on the inner surface of the outer layer (6) and/or of the barrier layer (9).

2. Garment according to claim 1, characterised in that intermediate portions (4A₂, 4B₂) of the elastic leg portions are disposed in the centre beneath a suction core (8), without being connected to one of the layers.

3. Garment according to claim 1 or 2, wherein the barrier layer (9) is disposed sandwich fashion between the outer and the inner layer (6, 7), substantially over its entire surface, and in which, in the forward and rear zones, the barrier layer (9) is provided along both lateral edges with air openings (16).

4. Garment according to at least one of claims 1 to 3, wherein the barrier layer (9) is narrower than the width of the outer and inner layers (6, 7).

5. Garment according to at least one of claims 1 to 4, wherein the barrier layer (9) is attached to the inner surface of the inner layer (7), a suction cure (8) is disposed sandwich fashion between the barrier layer (9) and the outer layer (6), and the core is attached at intermittent points at least either to the barrier layer (9) or to the inner layer (7).

6. Garment according to at least one of claims 1 to 5, wherein a laminate structure produced by the barrier layer (9) which is attached sandwich fashion between the outer and inner layers (6, 7) and is attached at intermittent points to one of the two layers, is folded along a longitudinal medial portion, and is attached together along the two lateral edges of the forward and rear zones, so that leg openings and a hip opening are defined, with which the elastic portions are associated.

7. Garment according to at least one of claims 1 to 6, wherein the outer and the inner layer (6, 7) are made from a fibre fleece, and the barrier layer (9) is made from an elastomeric film.

8. Garment according to at least one of claims 2 to 7, wherein the suction core (8) includes a mixture of heat-fused flocculent pulp, water-absorbent polymer particles and weldable fibres.

## Revendications

1. Pièce d'habillement comportant une couche supérieure élastique et perméable à l'eau (6), une couche inférieure élastique et perméable à l'eau (7), et des éléments élastiques de serrage des jambes et de la taille (4, 5), respectivement prévus sur les deux côtés, dans la région de l'entrejambe et dans les extrémités correspondantes de zones avant et arrière, une couche de barrage élastique (9) prise en sandwich entre les couches supérieure et inférieure (6, 7) et fixée par intermittence au moins à l'une des deux couches précitées, caractérisée en ce que chacun des éléments élastiques de serrage des jambes (4) présente une première portion partielle (4A) et une seconde portion partielle (4B), qui s'entrecoupent en des points situés à proximité de leurs extrémités opposées, et dont les portions latérales (4A₁ , 4B₁ ) s'étendent des points d'intersection respectifs vers les extrémités respectives, et sont fixées à la surface intérieure de la couche supérieure (6) et/ou de la couche de barrage (9).

2. Pièce d'habillement suivant la revendication 1, caractérisée en ce que des portions intermédiaires (4A₂ , 4B₂ ) des éléments élastiques de serrage des jambes sont agencées dans la partie centrale, sous un corps central absorbant (8), sans être rattachées à l'une des couches.

3. Pièce d'habillement suivant l'une des revendications 1 ou 2, caractérisée en ce que la couche de barrage (9) est emprisonnée en sandwich entre les couches supérieure et inférieure (6, 7), pour ainsi dire sur toute leur étendue, et en ce que dans les zones avant et arrière, la couche de barrage (9) est pourvue d'orifices d'aération (18) le long des deux bords latéraux.

4. Pièce d'habillement suivant au moins l'une des revendications 1 à 3, dans laquelle la couche de barrage (9) est plus étroite que la largeur des couches supérieure et inférieure (6, 7).

5. Pièce d'habillement suivant au moins l'une des revendications 1 à 4, dans laquelle la couche de barrage (9) est fixée à la surface interne de la couche inférieure (7), un corps central absorbant (8) est pris en sandwich entre la couche de barrage (9) et la couche supérieure (6), et dans laquelle le corps central est fixé de manière intermittente au moins à la couche de barrage (9) ou à la couche inférieure (7).

6. Pièce d'habillement suivant au moins l'une des revendications 1 à 5, dans laquelle une structure lamifiée, fabriquée à partir de la couche de barrage (9) prise en sandwich entre les couches supérieure et inférieure (6, 7) et fixée par intermittence à l'une des deux couches, est pliée en suivant une portion longitudinale médiane et réunie en suivant les deux bords latéraux de la zone avant et arrière, ce qui a pour effet de définir les ouvertures de passage des jambes et une ouverture de passage des hanches, auxquelles sont associées les éléments élastiques.

7. Pièce d'habillement suivant au moins l'une des revendications 1 à 6, dans laquelle les couches supérieure et inférieure (6, 7) sont fabriquées à partir d'un non-tissé fibreux, et la couche de barrage (9) à partir d'un film élastomère.

8. Pièce d'habillement suivant au moins l'une des revendications 2 à 7, dans laquelle le corps central absorbant (8) comprend un mélange constitué par une pâte floconneuse thermofusible, par des particules polymères absorbant l'eau et par des fibres soudables.
